# FASCICULE DE BREVET EUROPEEN

(11) **EP 3 624 873 B1**
(45) Date de publication et mention de la délivrance du brevet: **01.11.2023**
(21) Numéro de dépôt: 18723879.5
(22) Date de dépôt: 18.05.2018
(51) Int. Cl.: A61M 5/00, A61M 5/178, A61M 5/168, A61K 51/12, A61M 5/145, A61M 5/14

(54) **INSTALLATION POUR L'INJECTION A UN PATIENT D'UN PRODUIT RADIOACTIF ET SON PROCEDE DE MISE EN OEUVRE**
INSTALLATION ZUR INJEKTION EINES RADIOAKTIVEN PRODUKTS IN EINEN PATIENTEN UND VERFAHREN ZU DESSEN DURCHFÜHRUNG
INSTALLATION FOR THE INJECTION OF A RADIOACTIVE PRODUCT INTO A PATIENT AND METHOD FOR THE IMPLEMENTATION THEREOF

(30) Priorité: 19.05.2017 FR 1754469
(43) Date de publication de la demande: 25.03.2020
(73) Titulaire: LEMER PAX, 44240 La Chapelle-sur-Erdre (FR)
(72) Inventeur: LEMER, Pierre-Marie, 44100 Nantes (FR); SETOAIN, Xavier, 08036 Barcelona (ES)
(74) Mandataire: Jacobacci Coralis Harle
(86) Numéro de dépôt international: PCT/EP2018/063158
(87) Numéro de publication internationale: WO 2018/211092

(56) Documents cités:
- WO-A1-99/56117
- WO-A2-2009/152320
- WO-A2-2010/039573
- FR-A1- 2 917 981
- US-A1- 2014 276 411

## Description

### DOMAINE TECHNIQUE AUQUEL SE RAPPORTE L'INVENTION

La présente invention concerne le domaine général de la médecine nucléaire. Elle concerne plus particulièrement une installation pour l'injection à un patient d'un produit radioactif.

### ARRIERE-PLAN TECHNOLOGIQUE

Certaines techniques d'imagerie médicale (notamment la tomographie par émission monophotonique TEMP, dite aussi SPECT) utilisent des substances radioactives émettrices de rayonnements ionisants, appelées radiopharmaceutiques ou radiotraceurs, qui sont constitués d'un radio-isotope associé à un vecteur adapté pour se fixer sélectivement dans l'organisme.

Après injection de ce radiotraceur au patient, on réalise l'image de sa distribution dans l'organisme, par exemple à l'aide d'une gamma-caméra.

Pour limiter les doses de radiations reçues par le patient et par le personnel chargé des manipulations, on utilise des radio-isotopes de courtes demi-vies à usage médical, c'est-à-dire dont le niveau de radiation émis décroît rapidement avec le temps.

Par exemple, on utilise de tels éléments radiotraceurs pour la recherche du foyer épileptogène chez les patients atteints d'affections épileptiques.

Pour cela on prépare à l'avance une seringue contenant l'agent de perfusion cérébrale SPECT choisi (notamment du 99mTc-HMPAO ou 99mTc-ECD), et un opérateur réalise l'injection dès l'apparition d'une crise épileptique.

Il est alors essentiel de réagir très vite et d'injecter très rapidement le produit (en quelques secondes), pour obtenir une fixation la plus localisée possible du radiotraceur sur le foyer épileptogène, et par voie de conséquence pour obtenir une image très précise de ce foyer, afin de confirmer la possibilité d'une intervention chirurgicale, d'une part, et de localiser au mieux la zone défaillante du cerveau à supprimer chirurgicalement, d'autre part.
De plus, idéalement, il convient d'injecter une activité (ou, par abus de langage, une dose) précise de ce radiotraceur, adaptée au patient, fonction de la sensibilité de la caméra et de l'organe humain à étudier, pour obtenir la qualité d'image juste nécessaire au bon diagnostic. A noter qu'une trop faible activité injectée peut mener à l'impossibilité de lire l'image obtenue et donc de devoir exécuter à nouveau l'injection. A contrario, une activité trop grande peut saturer la caméra utilisée (ce qui rend difficile le diagnostic également), mais surtout mène à une surexposition inutile du patient aux rayonnements ionisants.

Cependant les crises d'épilepsie apparaissant de manière assez aléatoire, l'urgence à intervenir ne permet pas à l'opérateur de réaliser le calcul d'un volume précis à injecter, tenant compte de l'activité restante dans la seringue à ce moment (du fait de la décroissance rapide d'activité du radio-isotope).
C'est pourquoi, de manière habituelle, l'opérateur injecte la totalité du volume de la seringue à sa disposition, avec les risques inhérents en terme de surexposition du patient et de qualité d'image.

En outre, malgré le type de produit mis en oeuvre (courte demi-vie), une autre contrainte à prendre en compte concerne la radioprotection du personnel médical chargé de préparer la dose du radiotraceur et de l'injecter au patient.

De manière classique, les doses à injecter sont prélevées dans une seringue munie d'un blindage approprié, placée elle-même dans une enceinte blindée équipée de moyens de mesure et de contrôle appropriés, permettant de prélever dans la seringue la dose de produit radioactif recherchée. Ensuite, un opérateur récupère la seringue blindée et il se rend auprès du patient pour réaliser l'injection.

Cependant, cette manière d'opérer n'offre pas une sécurité optimale sur le plan de la radioprotection pour l'opérateur et même pour le patient.

Dans ce cadre, les documents US-6 767 319 et WO-2008-037939 décrivent des matériels de calibrage et d'injection de produit radioactif visant à limiter l'exposition du personnel à la substance radioactive et aussi à optimiser la sécurité du patient.

Les installations correspondantes comprennent - des moyens pour le support d'une source en produit radioactif injectable, - des moyens pour le support d'une seringue, qui sont équipés de moyens pour la manoeuvre automatique de son piston, et qui sont associés à un dispositif de type activimètre pour la mesure en temps réel de l'activité radio-isotopique émise par le produit contenu dans la seringue, et - un système de vannes qui est raccordé hydrauliquement, par le biais de tubulures, à la source mère radioactive, à la seringue, à une source de sérum physiologique et à un cathéter d'injection destiné à être connecté au patient.

La source de produit radioactif et la seringue, notamment, sont entourées de matériau radio-protecteur.

Ces matériels comprennent encore des moyens destinés à piloter le système de vannes et les moyens de manoeuvre du piston de seringue, cela de manière adaptée pour assurer, dans un premier temps, le prélèvement d'une dose de produit radioactif et/ou de sérum physiologique au sein de la seringue, et dans un second temps l'éjection, au travers du cathéter d'injection, du produit radioactif et/ou du sérum physiologique préalablement prélevés. La dose de produit radioactif est mesurée par le dispositif activimètre au cours du prélèvement dans la seringue.

Mais de telles installations sont relativement complexes ; en outre elles ne permettent pas une injection rapide du produit au patient, notamment du fait des moyens utilisés pour la manoeuvre du piston de seringue.

Les documents FR-2 917 981, WO-2010/039573 et US-2014/276411 décrivent encore des installations du même type. Là encore, les moyens de manoeuvre des pistons de seringue consistent en des actionneurs ou motorisations classiques, qui ne permettent pas une injection rapide du produit au patient.

Les documents WO-2009/152320 et WO-99/56117 proposent encore d'autres installations pour l'injection d'un produit à un patient, dans lesquelles des pistons de seringues sont associés à des motorisations de manoeuvre.

### OBJET DE L'INVENTION

Afin de remédier à l'inconvénient précité de l'état de la technique, la présente invention propose une installation d'injection à un patient d'un produit radioactif, laquelle installation est du type comprenant :
- une première seringue comprenant un corps muni d'un embout et un piston, lequel embout est relié à un moyen d'injection au patient par l'intermédiaire d'une tubulure d'injection, lequel corps de première seringue est adapté pour contenir ledit produit radioactif à injecter,
- des moyens de manoeuvre dudit piston de première seringue,
- des moyens pour déterminer en permanence ou de manière régulière le volume de produit radioactif à injecter au patient, tenant compte d'une activité prescrite à injecter au patient,
- des moyens de commande de déclenchement pour le déclenchement de l'injection du produit radioactif au patient,
- une seconde seringue, comprenant un corps muni d'un embout et un piston, lequel corps de seconde seringue est adapté pour contenir un produit de rinçage,
et lequel embout de seconde seringue est relié par un canal audit embout de ladite première seringue ou à une partie amont de ladite tubulure d'injection,
cette installation étant caractérisée par le fait qu'elle comprend :
- ladite première seringue d'axe longitudinal vertical, avec son piston orienté vers le haut,
- lesdits moyens de manoeuvre dudit piston de ladite première seringue, comprenant une masse d'injection, apte à être positionnée au dessus dudit piston de ladite première seringue, et apte à venir appuyer sur ledit piston par gravité, pour assurer l'injection du produit radioactif au patient,
laquelle masse d'injection est associée à des moyens de maintien désactivables, qui permettent son maintien en position inactive empêchant ladite injection, et sa libération pour assurer ledit appui sur ledit piston de ladite première seringue,
- une structure de butée mobile manoeuvrée par une motorisation de butée, laquelle structure de butée mobile est agencée pour coopérer avec ledit piston de ladite première seringue et/ou avec ladite masse d'injection, de manière, à partir d'une position dudit piston extraite dudit corps de première seringue, à constituer une butée de fin de course en rentrée dudit piston, en fonction du volume de produit radioactif à injecter au patient,
- des moyens pour détecter l'atteinte de la fin de course dudit piston de ladite première seringue, par rapport à ladite structure de butée mobile,
- ladite seconde seringue d'axe longitudinal vertical, avec son piston orienté vers le haut,
- des moyens de manoeuvre dudit piston de ladite seconde seringue, comprenant une masse de rinçage, apte à être positionnée au dessus dudit piston de ladite seconde seringue, et apte à venir appuyer sur ledit piston par gravité, pour assurer le rinçage au moins partiel de ladite tubulure,
laquelle masse de rinçage est associée à des moyens de maintien désactivables, qui permettent son maintien en position inactive empêchant ledit rinçage, et sa libération pour assurer ledit appui sur ledit piston de ladite seconde seringue,
- des moyens de gestion comprenant :
- des moyens pour commander ladite motorisation de butée afin de déplacer ladite structure de butée mobile en fonction de ladite activité prescrite à injecter au patient,
- des moyens pour commander lesdits moyens de maintien désactivables de ladite masse d'injection,
- des moyens pour commander lesdits moyens de maintien désactivables de ladite masse de rinçage.

Une telle combinaison de caractéristiques permet une injection rapide et sûre du produit radioactif.

D'autres caractéristiques non limitatives et avantageuses de l'installation conforme à l'invention, prises individuellement ou selon toutes les combinaisons techniquement possibles, sont les suivantes :
- lesdits moyens de maintien désactivables de ladite masse d'injection et lesdits moyens de maintien désactivables de ladite masse de rinçage consistent en des électroaimants, et lesdites masses d'injection et de rinçage sont réalisées en matériau approprié pour permettre leur maintien et leur libération par lesdits électroaimants ;
- l'installation comprend des moyens pour détecter la fin de course dudit piston de ladite seconde seringue ;
- l'installation comprend un clapet anti-retour en aval de l'embout de ladite première seringue, adapté pour empêcher le retour de fluide dans ladite première seringue, ainsi qu'un clapet anti-retour en aval de l'embout de ladite seconde seringue, adapté pour empêcher le retour de fluide dans ladite seconde seringue ;
- l'installation comprend :
   a/ une unité mobile montée sur roues comprenant :
      - ladite première seringue associée à ladite butée mobile manoeuvrée par ladite motorisation de butée, lesdits moyens de détection de fin de course de son piston, ladite masse d'injection et ses moyens de maintien désactivables, ladite tubulure d'injection et ledit moyen d'injection au patient,
      - ladite seconde seringue associée à ladite masse de rinçage et ses moyens de maintien désactivables,
      - des moyens de commande de déclenchement pour le déclenchement de l'injection du produit radioactif au patient, et
      - un automate de gestion adapté pour déterminer en permanence ou de manière régulière le volume de produit radioactif à injecter au patient, tenant compte d'une activité prescrite à injecter au patient, pour commander ladite motorisation de butée afin de déplacer ladite structure de butée mobile en fonction de ladite activité prescrite à injecter au patient, et pour commander lesdits moyens de maintien désactivables de ladite masse d'injection, ainsi que lesdits moyens de maintien désactivables de ladite masse de rinçage,
      - un écran de paramétrage et de visualisation de statut,
      - un réceptacle en matériau protecteur contre les rayonnements ionisants, dans lequel est placé au moins ladite première seringue, et
   b/ un boitier de commande à distance comprenant des moyens de commande de déclenchement pour le déclenchement de l'injection du produit radioactif au patient, et un écran de visualisation de statut.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue schématique d'une installation conforme à l'invention ;
- la figure 2 est une vue en perspective de la structure externe d'une forme de réalisation possible de l'installation illustrée sur la figure 1.

### Installation

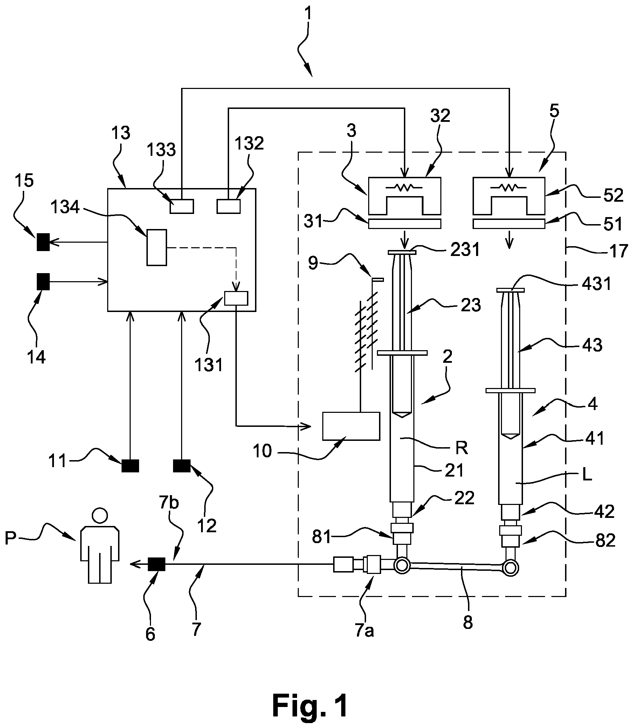

L'installation 1 conforme à l'invention représentée sur la figure 1 comprend - une première seringue 2 d'axe longitudinal vertical, comprenant un corps 21 muni d'un embout 22 et un piston 23 orienté vers le haut, laquelle première seringue 2 est associée à des moyens 3 de manoeuvre de son piston 23, et - une seconde seringue 4 d'axe longitudinal vertical, comprenant un corps 41 muni d'un embout 42 et un piston 43 orienté vers le haut, laquelle seconde seringue 4 est associée à des moyens 5 de manoeuvre de son piston 43.

Le corps 21 de la première seringue 2 est destiné à contenir un produit radioactif (radiotraceur) R que l'on souhaite injecter à un patient P ; et l'embout 22 de cette première seringue 2 est relié à un moyen 6 d'injection au patient par l'intermédiaire d'une tubulure d'injection 7. L'embout 22 de la première seringue 2 est raccordé à une partie amont 7a de la tubulure d'injection 7 ; et la partie aval 7b de cette tubulure d'injection 7 est raccordée au moyen 6 d'injection au patient.

Le corps 41 de seconde seringue 4 est adapté pour contenir un produit de rinçage L, et l'embout 42 de cette seconde seringue 4 est relié par un canal 8 à la partie amont 7a de la tubulure d'injection 7 (ou à l'embout 22 de ladite première seringue 2).

Les moyens 3 de manoeuvre du piston 23 de la première seringue 2 comprennent une masse d'injection 31 adaptée pour venir appuyer sur ledit piston 23 par gravité (plus précisément adaptée pour venir coopérer avec la tête 231 du piston 23), pour assurer l'injection du produit radioactif R au patient P ; cette masse d'injection 31 est associée à des moyens de maintien désactivables 32, qui permettent son maintien en position inactive empêchant ladite injection, et sa libération pour assurer ledit appui sur le piston 23 de ladite première seringue 2.

La masse d'injection 31 est maintenue par ses moyens de maintien désactivables 32 au dessus du piston 23 de la première seringue 2, à l'aplomb de celui-ci. En l'occurrence, cette masse d'injection 31 est ici maintenue par ses moyens de maintien désactivables 32 à distance du piston 23, séparée/écartée de ce dernier.

Ces moyens de maintien désactivables 32 se présentent sous la forme d'un électroaimant. La masse d'injection 31 est une pièce monobloc ou un assemblage de pièces, en matériau approprié (par exemple en inox) adapté pour permettre son maintien et sa libération par cet électroaimant 32.

Les moyens 5 de manoeuvre du piston 43 de la seconde seringue 4 comprennent une masse de rinçage 51 adaptée pour venir appuyer sur ledit piston 43 par gravité (plus précisément adaptée pour venir coopérer avec la tête 431 du piston 43), pour assurer le rinçage au moins partiel de la tubulure d'injection 7 ; cette masse de rinçage 51 est associée à des moyens de maintien désactivables 52, qui permettent son maintien en position inactive empêchant ledit rinçage, et sa libération pour assurer ledit appui sur ledit piston 43 de ladite seconde seringue 4.

La masse de rinçage 51 est maintenue par ses moyens de maintien désactivables 52 au dessus du piston 43 de la seconde seringue 4, à l'aplomb de celui-ci. En l'occurrence, cette masse de rinçage 51 est ici maintenue par ses moyens de maintien désactivables 52 à distance du piston 43, séparée/écartée de ce dernier.

Ces moyens de maintien désactivables 52 se présentent sous la forme d'un électroaimant. La masse de rinçage 51 est une pièce monobloc ou un assemblage de pièces, en matériau approprié (par exemple en inox) adapté pour permettre son maintien et sa libération par cet électroaimant 52.

Par exemple, les masses 31, 51 sont maintenues en place lorsque les électroaimants 32, 52 sont hors tension et elles ne sont lâchées que lorsque les électroaimants 32, 52 sont alimentés.

L'installation 1 comprend également une structure de butée mobile 9 manoeuvrée par une motorisation de butée 10, laquelle structure de butée mobile 9 est agencée pour coopérer avec le piston 23 de ladite première seringue 2 et/ou avec la masse d'injection 31, de sorte, à partir d'une position dudit piston 23 extraite dudit corps de première seringue 21, à constituer une butée de fin de course en rentrée dudit piston 23, en fonction du volume de produit radioactif R que l'on souhaite injecter au patient P.

Cette structure de butée mobile 9 coopère ici avec le dessous de la masse d'injection 31. Dans une variante de réalisation, elle peut coopérer avec le dessous de la tête 231 du piston 23 de la seringue 2.

La motorisation de butée 10 consiste en une table linéaire, un moteur et un réducteur pour améliorer la précision du déplacement.

L'installation 1 comporte encore des moyens 11 pour détecter l'atteinte de la fin de course du piston 23 de ladite première seringue 2, par rapport à ladite structure de butée mobile 9 ; ces moyens de détection 11 peuvent consister en une vis d'arrêt à contact électrique, coopérant avec la masse d'injection 31.

Elle comporte aussi des moyens 12 pour détecter l'atteinte de la fin de course du piston 43 de ladite seconde seringue 4, par rapport à son corps 41 ; ces moyens de détection 12 peuvent consister en une vis d'arrêt à contact électrique, qui coopère avec la masse de rinçage 51.

Le fonctionnement de l'installation 1 est géré par des moyens de gestion 13 en forme d'automate calculateur comprenant :
- des moyens 131 pour commander la motorisation de butée 10,
- des moyens 132 pour commander les moyens de maintien désactivables 32 de la masse d'injection 31,
- des moyens 133 pour commander les moyens de maintien désactivables 52 de la masse de rinçage 51,
- des moyens 134 pour déterminer en permanence ou de manière régulière le volume de produit radioactif R à injecter au patient, tenant compte d'une activité prescrite à injecter au patient.

Pour cela ces moyens de détermination 134 consistent en des moyens de calcul qui tiennent compte de l'activité initiale introduite dans le corps 21 de la première seringue 2, de la demi-vie du radio-isotope en présence et de la durée écoulée entre, d'une part, le moment du remplissage du corps de seringue 21 avec le produit radioactif R et, d'autre part, le moment de l'injection au patient.

Le calcul correspondant peut être réalisé en permanence ou bien de manière régulière, par exemple toutes les 30 secondes, toutes les minutes ou autre.

Partant de l'activité calculée présente dans le corps 21 de la seringue 2, l'automate 13 commande la motorisation de butée 10, en permanence ou de manière régulière, pour déplacer la structure de butée mobile 9 en fonction du volume de produit R à injecter au patient correspondant à l'activité prescrite.

Cela permet d'injecter au patient l'activité prescrite, quel que soit le moment de l'injection par rapport au moment initial de préparation de la seringue.

L'installation 1 comporte encore des moyens de commande de déclenchement 14 pour le déclenchement de l'injection du produit radioactif R au patient. Ces moyens de commande de déclenchement 14 consistent en au moins un bouton poussoir destiné à être actionné par un opérateur ; de préférence l'installation comporte deux boutons poussoirs destinés à être actionnés simultanément, pour éviter les déclenchements involontaires.

De préférence encore ces moyens de commande de déclenchement 14 sont doublés et comprennent :
- des premiers moyens de commande de déclenchement (avantageusement en forme de deux boutons poussoirs) ménagés sur une unité mobile intégrant les seringues d'injection 2 et 4, les moyens 3, 5 de commande de leurs pistons 23, 43 et la tubulure d'injection 7, cette unité mobile étant destinée à être placée à côté du patient P, et
- des seconds moyens de commande de déclenchement (également avantageusement en forme de deux boutons poussoirs), ménagés sur un boitier de commande à distance, permettant à un opérateur de commander le déclenchement de l'injection à distance du patient (et donc à distance de la source radioactive).

Des moyens de sécurité sont avantageusement prévus (gérés par l'automate 13) adaptés pour empêcher l'injection du radiotraceur R au patient, notamment dans le cas où l'activité restante dans le corps de seringue 21 est inférieure à l'activité prescrite pour le patient, ou encore si le produit R à injecter est considéré comme périmé.

L'installation comporte encore avantageusement une alarme sonore 15 adaptée pour être activée tout au long de l'opération d'injection, c'est-à-dire depuis l'ordre de commande d'injection jusqu'à la fin de l'injection du rinçage.

Comme on peut le voir sur la figure 1, un clapet anti-retour 81 est prévu en aval de l'embout 22 de la première seringue 2, adapté pour empêcher le retour de fluide en direction de la première seringue 2. De même, un clapet anti-retour 82 est prévu en aval de l'embout 42 de la seconde seringue 4 pour empêcher le retour de fluide en direction de la seconde seringue 4.

D'autre part, l'installation comporte des moyens pour protéger l'environnement et en particulier le ou les opérateurs et le patient, contre les rayonnements ionisants émis par le radiotraceur R.

Pour cela, au moins la première seringue 2 est placée dans un réceptacle 17 en matériau protecteur contre les rayonnements ionisants, par exemple un réceptacle en plomb de 4 mm d'épaisseur.

L'automate de gestion 13 est configuré et paramétré pour mettre en oeuvre les différentes fonctionnalités décrites ci-dessus. Il est associé à une interface homme-machine, avec écran de visualisation, adaptée pour entrer l'ensemble des paramètres nécessaires à sa mise en oeuvre, en fonction de l'application envisagée.

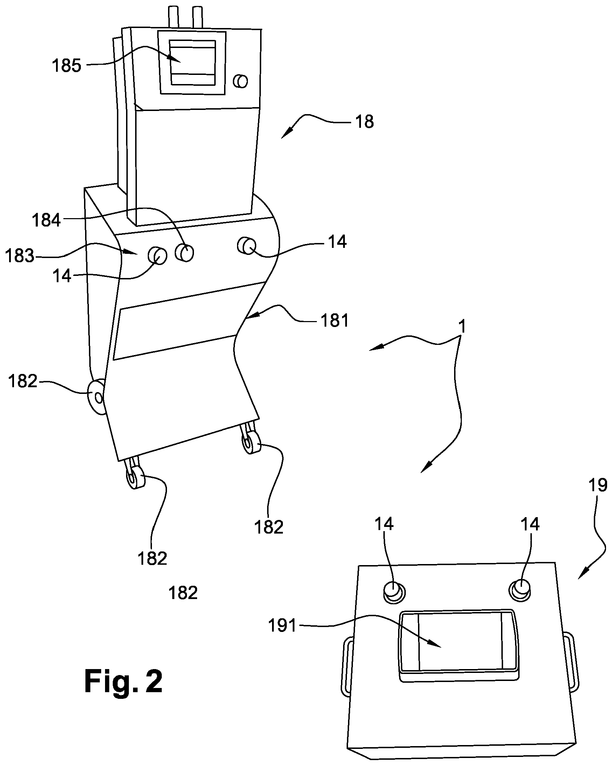

La figure 2 illustre une configuration structurelle possible de l'installation d'injection 1.

Dans cette forme de réalisation l'installation d'injection 1 comprend, d'une part, une unité mobile 18 et, d'autre part, un boitier de commande à distance 19.

L'unité mobile 18 consiste en une sorte de caisson 181 monté sur des roues 182, qui comprend l'ensemble des moyens pour assurer l'injection du radiotraceur R au patient P, ainsi que les moyens qui permettent la gestion sécurisée de cette injection.

Cette unité mobile 18 comprend donc différents matériels internes n'apparaissant pas sur la figure 2, a savoir : la première seringue 2 contenant le radiotraceur R, la butée mobile 9 manoeuvrée par la motorisation de butée 10, les moyens 11 de détection de fin de course du piston de seringue 23, la masse d'injection 31 et ses moyens de maintien désactivables 32, la seconde seringue 4 contenant le produit de rinçage L, la masse de rinçage 51 et ses moyens de maintien désactivables 52, au moins la seringue 2 étant placée dans un réceptacle radio-protégé (par exemple en plomb de 4 mm d'épaisseur).

Elle comprend encore :
- la tubulure d'injection 7 et le moyen d'injection 6 (par exemple une aiguille intraveineuse), non représentés,
- l'automate de gestion 13 (non visible),
- un pupitre de commande manuelle 183 avec deux boutons poussoirs 14 de commande pour le déclenchement de l'injection du produit radioactif R au patient et un bouton on/off 184,
- un écran 185 de paramétrage et de visualisation de statut.

De son côté, le boitier de commande à distance 19 comprend deux boutons poussoirs 14 de commande pour le déclenchement de l'injection du radiotraceur R au patient, et un écran de visualisation de statut 191.

### Procédé

L'installation 1 est particulièrement destinée à l'injection d'agents de perfusion cérébrale. Elle permet en particulier de calculer le volume de radiotraceur R pour injecter en quelques secondes (par exemple moins de 6 secondes) un agent de perfusion cérébrale pour la localisation d'un foyer épileptogène, tout en préservant les opérateurs de l'exposition aux rayonnements ionisants.

Le procédé pour la mise en oeuvre de cette installation 1 comprend les opérations suivantes :
a/ Réalisation des différents contrôles de l'état fonctionnel de l'installation, notamment de son alimentation électrique et du positionnement haut des masses d'injection 31 et de rinçage 51 (positionnement écarté des pistons de seringue 23, 43) ;
b/ Préparation de la première seringue 2 avec son corps de seringue 21 contenant le radiotraceur R approprié, par exemple 8 ml d'un mélange HMPAO + Tc99m d'une activité de 950MBq, et préparation de la seconde seringue 4 avec son corps de seringue 41 contenant le produit de rinçage L, par exemple 5 ml de Chlorure de Sodium (NaCl) ; ensuite positionnement respectif de ces deux seringues sous la masse d'injection 31 et sous la masse de rinçage 51, maintenues par leurs moyens de maintien respectifs 32, 52 ;
c/ Initialisation de l'installation par appui sur le bouton poussoir on/off 184 ;
d/ Vérification des paramètres généraux du matériel, notamment la date et l'heure du système ;
e/ Eventuellement authentification de l'opérateur par entrée d'un mot de passe ;
f/ Saisie des paramètres d'identification du patient (nom, prénom, date de naissance), des paramètres liés au radiotraceur R (activité préparée, nature du radiotraceur, date et heure de préparation) et des paramètres liés à la prescription (activité prescrite, nom du préparateur...) ;
g/ Une fois ces informations correctement renseignées, l'automate calculateur commande le déplacement de la structure de butée mobile 9 pour prendre sa position adaptée en fonction de l'activité prescrite, de l'activité préparée, la date de préparation et la date courante ;

Un chronomètre se met en route pour calculer l'activité restante dans la première seringue 2 et vérifier la péremption du produit ;

Par la suite, l'automate calculateur détermine en permanence ou de manière régulière le volume de produit radioactif R à injecter au patient, tenant compte de l'activité cible prescrite destinée à être injectée au patient, et il déplace en conséquence la structure de butée mobile 9 par la motorisation de butée 10 (par exemple toutes les 90 secondes) ;
h/ Dans l'hypothèse où la seringue 2 de radiotraceur R déjà chargée ne contient plus suffisamment d'activité, et/ou le radiotraceur R a dépassé sa date de péremption, le matériel en informe l'opérateur et il est alors nécessaire de remplacer le radiotraceur R et d'enregistrer les nouvelles données ;
i/ Dès l'apparition d'une crise chez le patient : actionnement par l'opérateur des moyens de commande de l'injection du radiotraceur R, par appui simultané, soit sur les deux boutons poussoirs 14 de l'unité mobile 18, soit (de préférence) sur les deux boutons poussoirs 14 du boitier de commande à distance 19 ;
j/ Les moyens de maintien désactivables 32 de la masse d'injection 31 sont désactivés pour la libérer et assurer son appui sur le piston 23 de la première seringue 2 afin d'alimenter la tubulure d'injection 7 et réaliser l'injection du radiotraceur R au patient P ;
k/ Après détection de la fin de course du piston 23 de la première seringue 2 (par exemple par contact de la masse d'injection 31 avec le détecteur 11 de fin de course d'injection), les moyens de maintien 52 de la masse de rinçage 51 sont désactivés, pour la libérer et assurer son appui sur le piston 43 de la seconde seringue 4 afin de réaliser le rinçage de la tubulure d'injection 7 et assurer l'injection de la totalité de l'activité prescrite au patient P ;

La fin de l'étape de rinçage est détectée par contact de la masse de rinçage 51 avec le détecteur 12 de fin de course de rinçage) ;
l/ Une fois l'injection réalisée, l'opérateur retourne à proximité de l'unité mobile pour utiliser les fonctions de sauvegarde et de remise à zéro. Les données d'injection et les données patient peuvent être sauvegardées sur un support externe ;
m/ Les masses d'injection et de rinçage 31, 51 doivent être replacées manuellement en position haute pour permettre le démarrage d'un nouveau cycle.

Au démarrage de l'injection du radiotraceur R et au démarrage de l'injection du produit de rinçage, des chronomètres se mettent en marche, stoppés lors des détections de fin de course des pistons 23, 43, pour connaitre les temps d'injection. Des alarmes adaptées 15 sont également activées au cours de ces injections (une même alarme pour les injections de radiotraceur R et de produit de rinçage L, ou deux alarmes différentes).

Une telle structure d'installation permet une injection très rapide de radiotraceur au patient (de l'ordre de quelques secondes, par exemple en moins de 6 secondes), et cela de manière très sécurisée, tant pour les opérateurs que pour les patients.

Une fois le radiotraceur R injecté au patient, il vient se fixer sur les zones les plus actives du cerveau et notamment la zone responsable de la crise d'épilepsie. Le personnel hospitalier dispose ensuite d'environ 3h pour emmener le patient en salle d'acquisition pour imager le cerveau à l'aide d'une gamma caméra.

## Revendications

1. Installation pour l'injection à un patient (P) d'un produit radioactif (R), laquelle installation (1) comprend :
- une première seringue (2), comprenant un corps (21) muni d'un embout (22) et un piston (23), lequel embout (22) est relié à un moyen (6) d'injection au patient (P) par l'intermédiaire d'une tubulure d'injection (7), lequel corps de première seringue (23) est adapté pour contenir ledit produit radioactif (R) à injecter,
- des moyens (3) de manoeuvre dudit piston de première seringue (23),
- des moyens (13) pour déterminer en permanence ou de manière régulière le volume de produit radioactif (R) à injecter au patient (P), tenant compte d'une activité prescrite à injecter au patient (P),
- des moyens (14) de commande de déclenchement pour le déclenchement de l'injection du produit radioactif (R) au patient (P),
- une seconde seringue (4), comprenant un corps (41) muni d'un embout (42) et un piston (43), lequel corps (41) de seconde seringue (4) est adapté pour contenir un produit de rinçage (L),
et lequel embout (42) de seconde seringue (4) est relié par un canal (8) audit embout (22) de ladite première seringue (2) ou à une partie amont de ladite tubulure d'injection (7),
cette installation (1) étant **caractérisée par le fait qu'**elle comprend :
- ladite première seringue (2) d'axe longitudinal vertical, avec son piston (23) orienté vers le haut,
- lesdits moyens (3) de manoeuvre dudit piston (23) de ladite première seringue (2), comprenant une masse d'injection (31), apte à être positionnée au dessus dudit piston (23), et apte à venir appuyer sur ledit piston (23) par gravité, pour assurer l'injection du produit radioactif (R) au patient (P),
laquelle masse d'injection (31) est associée à des moyens (32) de maintien désactivables, qui permettent son maintien en position inactive empêchant ladite injection, et sa libération pour assurer ledit appui sur ledit piston (23) de ladite première seringue (2),
- une structure de butée mobile (9) manoeuvrée par une motorisation de butée (10), laquelle structure de butée mobile (9) est agencée pour coopérer avec ledit piston (23) de ladite première seringue (2) et/ou avec ladite masse d'injection (31), de manière, à partir d'une position dudit piston (23) extraite dudit corps de première seringue (21), à constituer une butée de fin de course en rentrée dudit piston (23), en fonction du volume de produit radioactif (R) à injecter au patient (P),
- des moyens (11) pour détecter l'atteinte de la fin de course dudit piston (23) de ladite première seringue (2), par rapport à ladite structure de butée mobile (9),
- ladite seconde seringue (4) d'axe longitudinal vertical, avec son piston (43) orienté vers le haut,
- des moyens (5) de manoeuvre dudit piston (43) de ladite seconde seringue (4), comprenant une masse de rinçage (51), apte à être positionnée au dessus dudit piston (43), et apte à venir appuyer sur ledit piston (43) par gravité, pour assurer le rinçage au moins partiel de ladite tubulure (7),
laquelle masse de rinçage (51) est associée à des moyens (52) de maintien désactivables, qui permettent son maintien en position inactive empêchant ledit rinçage, et sa libération pour assurer ledit appui sur ledit piston (43) de ladite seconde seringue (4),
- des moyens de gestion (13) comprenant :
- des moyens (131) pour commander ladite motorisation de butée (10) afin de déplacer ladite structure de butée mobile (9) en fonction de ladite activité prescrite à injecter au patient (P),
- des moyens (132) pour commander lesdits moyens de maintien désactivables (32) de ladite masse d'injection (31),
- des moyens (133) pour commander lesdits moyens de maintien désactivables (52) de ladite masse de rinçage (51).

2. Installation selon la revendication 1, **caractérisée en ce que** lesdits moyens de maintien désactivables (32) de ladite masse d'injection (31) et lesdits moyens de maintien désactivables (52) de ladite masse de rinçage (51) consistent en des électroaimants, et **en ce que** lesdites masses d'injection (31) et de rinçage (51) sont réalisées en matériau approprié pour permettre leur maintien et leur libération par lesdits électroaimants (32, 52).

3. Installation selon l'une quelconque des revendications 1 ou 2, **caractérisée en ce qu'**elle comprend des moyens (12) pour détecter la fin de course dudit piston (43) de ladite seconde seringue (4).

4. Installation selon l'une quelconque des revendications 1 à 3, **caractérisée en ce qu'**elle comprend un clapet anti-retour (81) en aval de l'embout (22) de ladite première seringue (2), adapté pour empêcher le retour de fluide dans ladite première seringue (2), ainsi qu'un clapet anti-retour (82) en aval de l'embout (42) de ladite seconde seringue (4), adapté pour empêcher le retour de fluide dans ladite seconde seringue (4).

5. Installation selon l'une quelconque des revendications 1 à 4, **caractérisée en ce qu'**elle comprend :
a/ une unité mobile (18) montée sur roues (182) comprenant :
- ladite première seringue (2) associée à ladite structure de butée mobile (9) manoeuvrée par ladite motorisation de butée (10), lesdits moyens (11) de détection de fin de course de son piston (23), ladite masse d'injection (31) et ses moyens de maintien désactivables (32), ladite tubulure d'injection (7) et ledit moyen d'injection au patient (6),
- ladite seconde seringue (4) associée à ladite masse de rinçage (51) et ses moyens de maintien désactivables (52),
- des moyens (14) de commande de déclenchement pour le déclenchement de l'injection du produit radioactif (R) au patient (P), et
- un automate de gestion (13) adapté pour déterminer en permanence ou de manière régulière le volume de produit radioactif (R) à injecter au patient (P), tenant compte d'une activité prescrite à injecter au patient (P), pour commander ladite motorisation de butée (10) afin de déplacer ladite structure de butée mobile (9) en fonction de ladite activité prescrite à injecter au patient (P), et pour commander lesdits moyens de maintien désactivables (32) de ladite masse d'injection (31), ainsi que lesdits moyens de maintien désactivables (52) de ladite masse d'injection (51),
- un écran (185) de paramétrage et de visualisation de statut,
- un réceptacle (17) en matériau protecteur contre les rayonnements ionisants, dans lequel est placé au moins ladite première seringue (2), et
b/ un boitier (19) de commande à distance comprenant des moyens (14) de commande de déclenchement pour le déclenchement de l'injection du produit radioactif (R) au patient (P) et un écran de visualisation de statut (191).

## Patentansprüche

1. Installation zur Injektion eines radioaktiven Produkts (R) in einen Patienten (P), wobei die Installation
- eine erste Spritze (2) mit einem mit einem Endstück (22) versehenen Körper (21) und einem Kolben (23), wobei das Endstück (22) über einen Einspritzschlauch (7) mit einem Mittel (6) zum Einspritzen in den Patienten (P) verbunden ist, wobei der Körper (23) der ersten Spritze dazu ausgelegt ist, das einzuspritzende radioaktive Produkt (R) zu enthalten,
- Mittel (3) zum Betätigen des Kolbens (23) der ersten Spritze,
- Mittel (13) zum dauerhaften oder regelmäßigen Bestimmen des Volumens des in den Patienten (P) einzuspritzenden radioaktiven Produkts (R) unter Berücksichtigung einer in den Patienten (P) einzuspritzenden verschriebenen Aktivität,
- Auslösesteuermittel (14) zum Auslösen der Injektion des radioaktiven Produkts (R) in den Patienten (P),
- eine zweite Spritze (4) mit einem mit einem Endstück (42) versehenen Körper (41) und einem Kolben (43), wobei der Köper (41) der zweiten Spritze dazu ausgelegt ist, ein Spülmittel (L) zu enthalten,
und wobei das Endstück (42) der zweiten Spritze (4) über einen Kanal (8) mit dem Endstück (22) der ersten Spritze (2) oder einem stromaufwärtigen Teil des Einspritzschlauchs (7) verbunden ist,
aufweist,
wobei die Installation (1) durch die Tatsache gekennzeichnet ist, daß sie
- die erste Spritze (2) mit vertikaler Längsachse und dem nach oben gerichteten Kolben (23),
- die Mittel (3) zum Betätigen des Kolbens (23) der ersten Spritze (2) mit einer Einspritzmasse (31), die geeignet ist, über dem Kolben (23) positioniert zu werden, und geeignet ist, durch Schwerkraft auf den Kolben (23) zu drücken, um ein Einspritzen des radioaktiven Produkts (R) in den Patienten (P) sicherzustellen, wobei die Einspritzmasse (31) deaktivierbaren Haltemitteln (32) zugeordnet ist, die deren Halten in einer die Injektion verhindernden inaktiven Position und deren Freisetzung, um den Druck auf den Kolben (23) der ersten Spritze (2) sicherzustellen, ermöglichen,
- eine durch eine Anschlagsmotorisierung (10) gesteuerte mobile Anschlagsstruktur (9), wobei die Anschlagsstruktur (9) dazu ausgelegt ist, mit dem Kolben (23) der ersten Spritze (2) und/oder mit der Einspritzmasse (31) so zusammenzuwirken, daß sie von einer Position des Kolbens (23), in der er aus dem Körper (21) der ersten Spritze (21) ausgezogen ist, ausgehend, in Abhängigkeit vom Volumen des dem Patienten (P) einzuspritzenden radioaktiven Produkts (R), einen Anschlag als Laufwegende beim Einfahren des Kolbens (23) bildet,
- Mittel (11) zum Feststellen des Erreichens des Laufwegendes des Kolbens (23) der ersten Spritze (2) in Bezug auf die bewegliche Anschlagsstruktur (9),
- die zweite Spritze (4) mit senkrechter Längsachse und mit nach oben gerichtetem Kolben (43),
- Mittel (5) zum Betätigen des Kolbens (43) der zweiten Spritze (4) mit einer Spülmasse (51), die geeignet ist, über dem Kolben (43) positioniert zu werden, und geeignet ist, durch Schwerkraft auf den Kolben (43) zu drücken, um das mindestens teilweise Spülen der Schlauchleitung (7) sicherzustellen,
wobei die Spülmasse (51) deaktivierbaren Haltemitteln (52) zugeordnet ist, die deren Halten in einer die Spülung verhindernden inaktiven Position und deren Freisetzung, um den Druck auf den Kolben (43) der zweiten Spritze (4) sicherzustellen, ermöglichen,
- Organisationsmittel (13) mit
- Mitteln (131) zum Steuern der Anschlagsmotorisierung (10), um die mobile Anschlagsstruktur (9) in Abhängigkeit von der dem Patienten (P) einzuspritzenden verschriebenen Aktivität zu verlagern,
- Mitteln (132) zum Steuern der deaktivierbaren Mittel (32) zum Halten der Injektionsmasse (31),
- Mitteln (133) zum Steuern der deaktivierbaren Mittel (52) zum Halten der Spülmasse (51)
aufweist.

2. Installation gemäß Anspruch 1, **dadurch gekennzeichnet, daß** die deaktivierbaren Mittel (32) zum Halten der Injektionsmasse (31) und die deaktivierbaren Mittel (52) zum Halten der Spülmasse (51) aus Elektromagneten bestehen und daß die Injektionsmasse (31) und die Spülmasse (51) aus einem Material sind, das geeignet ist, deren Halten und Freigeben durch die Elektromagnete (32, 52) zu ermöglichen.

3. Installation gemäß einem der Ansprüche 1 oder 2, **dadurch gekennzeichnet, daß** sie Mittel (12) zum Erfassen des Laufwegendes des Kolbens (43) der zweiten Spritze (4) aufweist.

4. Installation gemäß einem der Ansprüche 1 bis 3, **dadurch gekennzeichnet, daß** sie stromabwärts vom Endstück (22) der ersten Spritze (2) eine Rückschlagklappe (81), die dazu ausgelegt ist, den Flüssigkeitsrückfluß in die erste Spritze (2) zu verhindern, und stromabwärts vom Endstück (42) der zweiten Spritze (4) eine Rückschlagklappe (82), die dazu ausgelegt ist, den Flüssigkeitsrückfluß in die zweite Spritze (4) zu verhindern, aufweist.

5. Installation gemäß einem der Ansprüche 1 bis 4, **dadurch gekennzeichnet, daß** sie
a) eine auf Rädern (182) montierte mobile Einheit (18) mit:
- der der durch eine Anschlagsmotorisierung (10) gesteuerten mobilen Anschlagsstruktur (9) zugeordneten ersten Spritze (2), den Mitteln (11) zum Erfassen des Laufwegendes des Kolbens (23), der Injektionsmasse (31) und deren deaktivierbare Haltemittel (32), dem Einspritzschlauch (7) und den Mitteln (6) zum Einspritzen in den Patienten,
- der der Spülmasse (51) zugeordneten zweiten Spritze (4) und deren deaktivierbaren Haltemitteln (52),
- den Mitteln (14) zum Steuern des Auslösens für das Auslösen der Injektion des radioaktiven Produkts (R) in den Patienten (P) und
- einem Organisationsautomaten (13), der dazu ausgelegt ist, dauerhaft oder regelmäßig das Volumen des dem Patienten (P) einzuspritzenden radioaktiven Produkts (R) zu bestimmen und dabei eine dem Patienten (P) zu verabreichende vorgeschriebene Aktivität zu berücksichtigen, um die Anschlagsmotorisierung (10) zu steuern, um die bewegliche Anschlagsstruktur (9) in Abhängigkeit von der dem Patienten (P) einzuspritzenden vorgeschrieben Tätigkeit zu verlagern, und zum Steuern der deaktivierbaren Haltemittel (32) der Einspritzmasse (31), sowie der deaktivierbaren Haltemittel (52) der Einspritzmasse (51),
- einem Schirm (185) zum Parametrieren und Sichtbarmachen des Status,
- einem Behälter (17) aus einem Material zum Schutz gegen ionisierende Strahlung, in dem mindestens die erste Spritze (2) angeordnet ist, und
b) einem Fernsteuergehäuse (19) mit Auslösesteuermitteln (14) zum Auslösen des Einspritzens des radioaktiven Einspritzprodukts (R) in den Patienten (P) und einem Anzeigeschirm (191) für den Status.

## Claims

1. Installation for injecting to a patient (P) a radioactive product (R), said installation (1) comprises:
- a first syringe (2), comprising a body (21) provided with a tip (22) and a plunger (23), said tip (22) being connected to a patient injection means (6) through an injection pipe (7), said first syringe body (21) being adapted to contain said radioactive product (R) to be injected,
- means (3) for operating said first syringe plunger (23),
- means (13) for determining continuously or on a regular basis the volume of radioactive product (R) to be injected to the patient (P), taking into account a prescribed activity to be injected to the patient (P),
- trigger control means (14) for triggering the injection of the radioactive product (R) to the patient (P),
- a second syringe (4), comprising a body (41) provided with a tip (42) and a plunger (43), said body (41) of second syringe (4) being adapted to contain a rinsing product (L),
and said tip (42) of second syringe (4) being connected by a channel (8) to said tip (22) of said first syringe (2) or to an upstream portion of said injection pipe (7),
said installation (1) being **characterized in that** it comprises:
- said first syringe (2) of vertical longitudinal axis, with its plunger (23) directed upward,
- said means (3) for operating said plunger (23) of said first syringe (2), comprising an injection mass (31), adapted to be positioned above said plunger (23) and capable of coming to press on said plunger (23) by gravity, to ensure the injection of the radioactive product (R) to the patient (P),
said injection mass (31) being associated with deactivatable holding means (32), which allow the holding thereof in inactive position for preventing said injection, and the release thereof to ensure said pressing on said plunger (23) of said first syringe (2),
- a mobile stop structure (9) operated by a stop motorization (10), said mobile stop structure (9) being arranged to cooperate with said plunger (23) of said first syringe (2) and/or with said injection mass (31), so as, from a position of said plunger (23) extracted from said first syringe body (21), to constitute an end-of-travel stop for the retraction of said plunger (23), as a function of the volume of radioactive product (R) to be injected to the patient (P),
- means (11) for detecting that said plunger (23) of said first syringe (2) has reached the end of travel, with respect to said mobile stop structure (9),
- said second syringe (4) of vertical longitudinal axis, with its plunger (43) directed upward,
- means (5) for operating said plunger (43) of said second syringe (4), comprising a rinsing mass (51), adapted to be positioned above said plunger (43), and capable of coming to press on said plunger (43) by gravity, to ensure the at least partial rinsing of said pipe (7),
said rinsing mass (51) being associated with deactivatable holding means (52), which allow the holding thereof in inactive position for preventing said rinsing, and the release thereof to ensure said pressing on said plunger (43) of said second syringe (4),
- management means (13) comprising:
- means (131) for controlling said stop motorization (10) in order to displace said mobile stop structure (9) as a function of said prescribed activity to be injected to the patient (P),
- means (132) for controlling said deactivatable holding means (32) of said injection mass (31),
- means (133) for controlling said deactivatable holding means (52) of said rinsing mass (51).

2. Installation according to claim 1, **characterized in that** said deactivatable holding means (32) of said injection mass (31) and said deactivatable holding means (52) of said rinsing mass (51) consist of electromagnets, and **in that** said injection (31) and rinsing (51) masses are made of a suitable material to allow the holding and releasing thereof by said electromagnets (32, 52).

3. Installation according to any one of claims 1 or 2, **characterized in that** it comprises means (12) for detecting that said plunger (43) of said second syringe (4) has reached the end of travel.

4. Installation according to any one of claims 1 to 3, **characterized in that** it comprises a check valve (81) downstream from the tip (22) of said first syringe (2), adapted to prevent the backflow of fluid into said first syringe (2), as well as a check valve (82) downstream from the tip (42) of said second syringe (4), adapted to prevent the backflow of fluid into said second syringe (4).

5. Installation according to any one of claims 1 to 4, **characterized in that** it comprises:
a/ a mobile unit (18) mounted on wheels (182) comprising:
- said first syringe (2) associated with said mobile stop structure (9) operated by said stop motorization (10), said means (11) for detecting the end of travel of its plunger (23), said injection mass (31) and the deactivatable holding means (32) thereof, said injection pipe (7) and said patient injection means (6),
- said second syringe (4) associated with said rinsing mass (51) and the deactivatable holding means (52) thereof,
- trigger control means (14) for triggering the injection of the radioactive product (R) to the patient (P), and
- a management automaton (13) adapted for determining continuously or on a regular basis the volume of radioactive product (R) to be injected to the patient (P), taking into account a prescribed activity to be injected to the patient (P), for controlling said stop motorization (10) in order to displace said mobile stop structure (9) as a function of said prescribed activity to be injected to the patient (P), and for controlling said deactivatable holding means (32) of said injection mass (31), as well as said deactivatable holding means (52) of said rinsing mass (51),
- a status setting and display screen (185),
- a receptacle (17) made of an ionizing radiation protective material, in which is placed at least said first syringe (2), and
b/ a remote-control box (19) comprising trigger control means (14) for triggering the injection of the radioactive product (R) to the patient (P), and a status display screen (191).
